# EUROPÄISCHE PATENTSCHRIFT

(11) **EP 1 827 531 B1**
(45) Veröffentlichungstag und Bekanntmachung des Hinweises auf die Patenterteilung: **14.07.2010**
(21) Anmeldenummer: 05789513.8
(22) Anmeldetag: 26.09.2005
(51) Int. Cl.: A61M 1/10

(54) **FALTBARE INTRAVASAL EINFÜHRBARE BLUTPUMPE**
FOLDING, INTRAVASCULARLY INSERTED BLOOD PUMP
POMPE A SANG PLIANTE, POUVANT ETRE INTRODUITE PAR VOIE INTRAVASCULAIRE

(30) Priorität: 12.11.2004 DE 102004054714
(43) Veröffentlichungstag der Anmeldung: 05.09.2007
(73) Patentinhaber: Abiomed Europe GmbH, 52074 Aachen (DE)
(72) Erfinder: SIESS, Thorsten, 52146 Würselen (DE)
(74) Vertreter: von Kreisler Selting Werner
(86) Internationale Anmeldenummer: PCT/EP2005/054804
(87) Internationale Veröffentlichungsnummer: WO 2006/051023

(56) Entgegenhaltungen:
- WO-A-94/05347
- WO-A-99/44651
- US-B1- 6 217 541

## Beschreibung

Die Erfindung betrifft eine faltbare intravasal einführbare Blutpumpe mit einem Rotor mit Flügeln, einer durch einen Katheter verlaufenden flexiblen Welle zum Antreiben des Laufrades und einer das Laufrad umschließenden Umhüllung.

Bekannt sind rotatorische Blutpumpen, die zur Unterstützung der Pumpleistung des natürlichen Herzens in das Herz eingeführt werden. Das Einführen geschieht intravasal, nämlich durch das Blutgefäßsystem des Patienten. Dies setzt voraus, dass der maximale Durchmesser der Blutpumpe beim Einführen nicht größer ist als 3 mm, sofern die Einführung über eine Schleuse und möglichst komplikationsarm erfolgen soll. Außerdem sollte die Blutpumpe möglichst flexibel sein, um sich Krümmungen des Gefäßverlaufes anzupassen.

Eine flexible intravasal einführbare Blutpumpe, von der der Oberbegriff des Anspruchs 1 ausgeht, ist beschrieben in WO 99/44651. Die Blutpumpe ist selbst entfaltbar und hat eine flexible komprimierbare Umhüllung in Form eines Rohres, das das Pumpengehäuse bildet. In der Umhüllung befindet sich ein radial komprimierbarer Rotor. Der Rotor ist nach Art einer Wendel ausgebildet, die die Blutströmung in axialer Richtung antreibt. Die Antriebswelle des Rotors verläuft durch einen Katheter hindurch. Der Katheter kann zusammen mit dem Gehäuse und dem Rotor in einen Deckschlauch hineingezogen werden. Bei einer derartigen Axialpumpe sind die Anforderungen an die Formgenauigkeit der Axialpumpe relativ hoch. Der Rotor muss der Innenform des Gehäuses mit engen Toleranzen folgen, um einerseits die Fördermenge von mindestens 2 l/min bei physiologisches Druckverhältnissen zu erreichen, ohne dabei andererseits Blut in zu starkem Maße zu zerstören. Diese Anforderungen sind bei einer faltbaren Blutpumpe nur schwer zu erfüllen. Die Axialpumpe ist mit einer relativ hohen Drehzahl von 30.000 bis 35.000 U/min zu betreiben.

Der Erfindung liegt die Aufgabe zugrunde, eine faltbare intravasal einführbare Blutpumpe mit vereinfachtem Aufbau und mit verringerter Störanfälligkeit zu schaffen.

Diese Aufgabe wird gelöst durch die Blutpumpe mit den Merkmalen des Patentanspruchs 1. Der Rotor ist ein radial förderndes Laufrad. Die Umfüllung im Bereich des Laufrades weist eine ringförmige Ausbauchung auf, wobei zwischen den radial äußeren Enden der Flügel und der Umhüllung ein ringförmiger Umlenkkanal gebildet ist, der die radiale Blutströmung in axialer Richtung umlenkt. Die erfindungsgemäße Blutpumpe ist eine Zentrifugalpumpe, bei der das Laufrad die Flüssigkeit ohne wesentliche axiale Komponente radial nach außen beschleunigt. Eine solche Zentrifugalpumpe benötigt keine engen Toleranzen zwischen Laufrad und Umhüllung. Die axiale Ausrichtung der Strömung wird durch die Umlenkung erzeugt. Die Blutpumpe ist unempfindlich gegen Fehlausrichtungen und ermöglicht große Toleranzen, was gerade bei einer faltbaren Blutpumpe von großer Bedeutung ist. Ein radial förderndes Laufrad benötigt zur Förderung einer typischen Blutmenge von etwa 2 bis 5 I pro Minute eine relativ geringe Drehzahl von etwa 5.000 bis 15.000 U/min. Der technische Aufbau der Radialpumpe ist einfach.

Das Laufrad und die Umhüllung können durch relatives Verschieben von Welle und Katheter faltbar sein. Der Vorgang des Faltens bzw. Entfaltens kann z. B. von dem Chirurgen oder Kardiologen beliebig aktiv eingeleitet werden.

Das Laufrad kann zwei im Wesentlichen parallele Stützwände haben, zwischen denen flexible Segel als Flügel ausgebildet sind. Dadurch ist ein technisch einfacher Aufbau eines faltbaren Laufrades gegeben, der bei rotierendem Laufrad die zur Blutförderung nötige Stabilität besitzt. Zur weiteren Erhöhung der Stabilität kann mindestens eine der Stützwände Speichen enthalten. Die Speichen können radial angeordnet sein und zusammen mit dem Laufrad an die Welle geklappt werden.

Eine Stützwand kann durchgehend sein, d. h. radial durchgehend von der Welle bis zum radial äußeren Ende der Stützwand, und die andere Stützwand kann eine die Welle umgebende Öffnung aufweisen. Die durchgehende Stützwand ist vorzugsweise die in Blutflussrichtung hintere Stützwand und die Stützwand mit der Öffnung ist vorzugsweise die in Blutflussrichtung vordere Stützwand. Das Blut kann durch die Stützwand mit der Öffnung einströmen und wird dann von der durchgehenden Stützwand in Richtung auf die Flügel umgelenkt. Dadurch ist eine einfache Form der Umlenkung eines axialen Blutstroms zur radialen Förderung ermöglicht.

Die Stützwände können mit einem Gelenk an der Welle befestigt sein. Die Stützwände sind dann zur Verringerung des Außendurchmessers der Blutpumpe zum Beispiel bei der intravasalen Einführung der Pumpe an die Welle klappbar. Durch die Wirkung der bei einer Rotation der Welle entstehenden Zentrifugalkraft besitzt das Laufrad die zur Blutförderung notwendige Stabilität.

Beide Stützwände können Speichen enthalten, die gegeneinander versetzt sind, so dass beim Klappen des Laufrades an die Welle die Speichen entlang des Umfangs der Welle nebeneinander liegen. Der Außendurchmesser der Blutpumpe in zusammengefaltetem Zustand ist dadurch gering.

Die beiden Stützwände können aus deckungsgleichen Speichen bestehen. Die Speichen bilden vorzugsweise Speichenräder, wobei jeweils zwischen den sich deckenden Speichen der beiden Speichenräder die flexiblen Segel angeordnet sind. Die Segel verlaufen durch die deckungsgleiche Anordnung der Speichen senkrecht zu der Ebene der Speichenräder. Durch diese Anordnung ist ein einfacher technischer Aufbau des Laufrades gegeben. Am vorderen Ende kann die Welle einen starren Teil aufweisen, an dem das Laufrad befestigt ist, um die Formstabilität der Blutpumpe zu erhöhen.

Die Umhüllung kann einen zylindrischen Fortsatz haben, an dessen Ende Durchströmöffnungen vorgesehen sind. Vorzugsweise kann das Blut durch die Durchströmöffnungen aus der Blutpumpe ausströmen bzw. bei Betrieb der Blutpumpe in entgegengesetzter Richtung kann das Blut durch die Durchströmöffnungen in die Blutpumpe einströmen. Die Umhüllung kann eine Nabe enthalten, z. B. an dem dem zylindrischen Fortsatz gegenüberliegenden Ende, in der die Welle axial unverschiebbar gelagert ist. Die Umhüllung ist dann an der Welle befestigt, ohne sich mit der Welle mitzudrehen.

Die Umhüllung kann eine konzentrische ebene Wand aufweisen, die in geringem Abstand von dem Laufrad angeordnet ist. Vorzugsweise ist die konzentrische ebene Wand in Blutflussrichtung vor dem Laufrad angeordnet. Durch den geringen Abstand zu dem Laufrad ist ein unerwünschter Blutrückfluss zwischen Laufrad und der Umhüllung gering. Die konzentrische ebene Wand weist vorzugsweise eine die Welle umgebende Öffnung auf, durch die das Blut in das Laufrad einströmen kann.

Das Laufrad kann in zwei einander entgegengesetzten Richtungen axial zu der Welle abklappbar sein. Die Umhüllung kann auf einer Seite des Laufrades eine Trennwand mit einer Durchlassöffnung im Bereich des Umlenkkanals aufweisen. Durch die Trennwand kann der axial in die Blutpumpe eintretende Blutfluss radial in Richtung auf die Flügel des Laufrades umgelenkt werden. Das von den Flügeln geförderte Blut kann durch die Durchlassöffnung ausströmen.

Im folgenden werden anhand der Zeichnungen Ausführungsbeispiele der Erfindung näher erläutert.

Es zeigen:
- Fig. 1: einen seitlichen Schnitt einer faltbaren intravasal einführbaren Blut- pumpe,
- Fig. 2: eine perspektivische Ansicht des Laufrades in Fig. 1,
- Fig. 3: eine Ansicht aus Richtung des Pfeiles III in Fig. 2,
- Fig. 4: einen Schnitt entlang der Linie IV-IV in Fig. 3,
- Fig. 5: die Blutpumpe nach Fig. 1 in teilweise zusammengefaltetem Zu- stand,
- Fig. 6: einen seitlichen Schnitt eines Ausführungsbeispiels der Blutpumpe mit abgewinkeltem Laufrad,
- Fig. 7: einen seitlichen Schnitt eines Ausführungsbeispiels der Blutpumpe mit gegenüber Fig. 6 in entgegengesetzter Richtung angewinkeltem Laufrad und entgegengesetzter Förderrichtung,
- Fig. 8: einen seitlichen Schnitt eines Ausführungsbeispiels der Blutpumpe mit einer zusätzlichen Trennwand hinter dem Laufrad,
- Fig. 9: eine Draufsicht auf das Laufrad des Ausführungsbeispiels nach Fig. 8,
- Fig. 10: ein erstes Anwendungsbeispiel der Blutpumpe im menschlichen Her- zen,
- Fig. 11: ein zweites Anwendungsbeispiel der Blutpumpe im menschlichen Herzen, und
- Fig. 12: ein drittes Anwendungsbeispiel der Blutpumpe im menschlichen Her- zen.

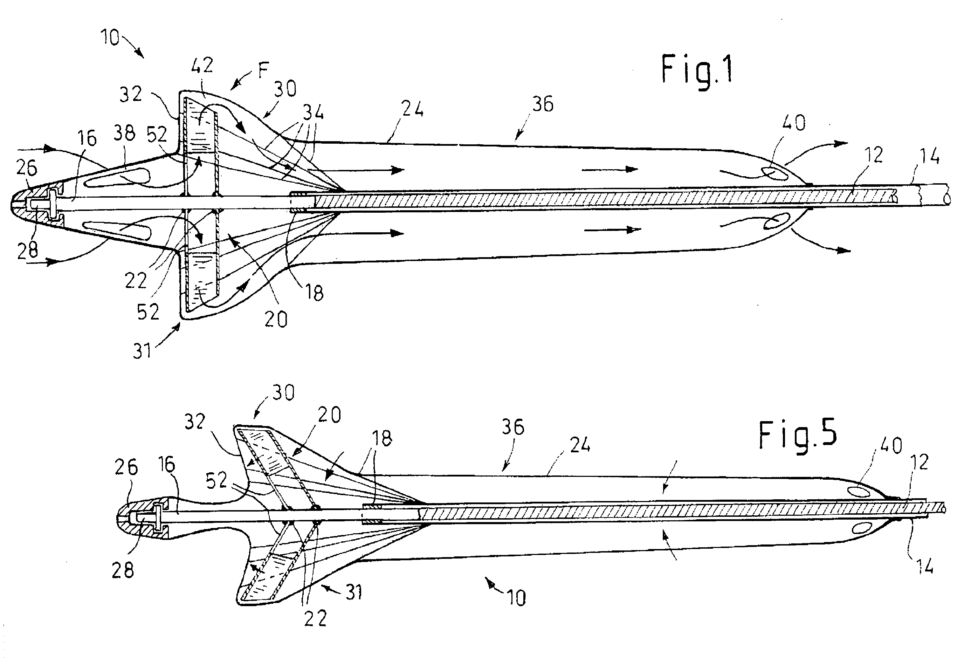

Die Blutpumpe 10 enthält eine langgestreckte flexible biegsame Welle 12, die in einem Katheter 14 enthalten ist. Der Katheter 14 besteht aus einem flexiblen Schlauch mit einem Durchmesser von etwa 2 mm. Der Katheter 14 besteht vorzugsweise aus einem abrasionsfesten Polymer wie Polyamid oder Polyurethan. Durch den Katheter 14 erstreckt sich die flexible Welle 12. Diese besteht in bekannter Weise aus einem multifilen Drahtbündel und kann hohl realisiert werden, um gegebenenfalls einen zentralen Führungsdraht 56 aufzunehmen. Am proximalen Ende wird die Welle 12 von einem nicht dargestellten Motor angetrieben, z. B. mit 5.000 bis 15.000 U/min, während der Katheter 14 festgehalten wird. An dem distalen, d. h. in Fig. 1 linken Ende, besitzt die Welle 12 einen starren Teil 16. Das distale Ende des Katheters 14 ist über ein Gleitlager 18 mit der Welle verbunden. Ein Laufrad 20 ist mit Gelenken 22 an dem starren Teil 16 der Welle 12 befestigt, so dass das Laufrad an die Welle 12 klappbar ist. Das Laufrad 20 und ein distaler Teil der Welle 12 und des Katheters 14 sind von einer Umhüllung 24 umschlossen. Die Umhüllung 24 besteht vorzugsweise aus einer sackartigen Polyurethanhaut. Aufgrund der Materialeigenschaften von Poly-urethan sind die Umhüllung 24 und der Katheter 14 gut miteinander verbindbar. Das distale Ende der Umhüllung 24 enthält eine Nabe 26, in der das distale Ende 28 der Welle 12 gehalten ist. Die Welle ist in der Nabe 26 nicht axial verschiebbar aber rotierbar, so das sich die Umhüllung 24 nicht mit der rotierenden Welle 12 mitdreht.

Die Umhüllung 24 hat im Bereich des Laufrades 20 eine ringförmige Ausbauchung 30, innerhalb der das Laufrad 20 rotiert. Der distale Teil der Umhüllung 24 mit der Ausbauchung 30 und dem Laufrad 20 bilden einen Pumpenkopf 31. Im distalen Bereich der Ausbauchung 30 enthält die Umhüllung 24 eine konzentrische ebene Wand 32, die mit einem geringen Abstand an der distalen Seite des Laufrades 20 angeordnet ist. Die Ausbauchung 30 ist durch konzentrisch angeordnete elastische Stege 34 verstärkt und aufgespannt. Die distalen Enden der Stege 34 sind mit der ebenen Wand 32 verbunden. Die proximalen Enden der Stege 34 sind mit dem Katheter 14 verbunden. Proximal der Ausbauchung 30 besitzt die Umhüllung 24 einen langgestreckten zylindrischen Fortsatz 36. Der Außendurchmesser des zylindrischen Fortsatzes 36 ist geringer als der der Ausbauchung 30. Im distalen und im proximalen Bereich hat die Umhüllung 24 vordere und hintere Durchströmöffnungen 38,40. In dem Ausführungsbeispiel in Fig. 1 ist die vordere Durchströmöffnung 38 eine Eintrittsöffnung und die hintere Durchströmöffnung 40 eine Austrittsöffnung. Zwischen dem radial äußeren Ende des Laufrades 20 und der Umhüllung 24 ist beim Betrieb der Blutpumpe ein ringförmiger Umlenkkanal 42 gebildet, durch den das radial geförderte Blut strömt und in Richtung auf die Welle umgelenkt wird.

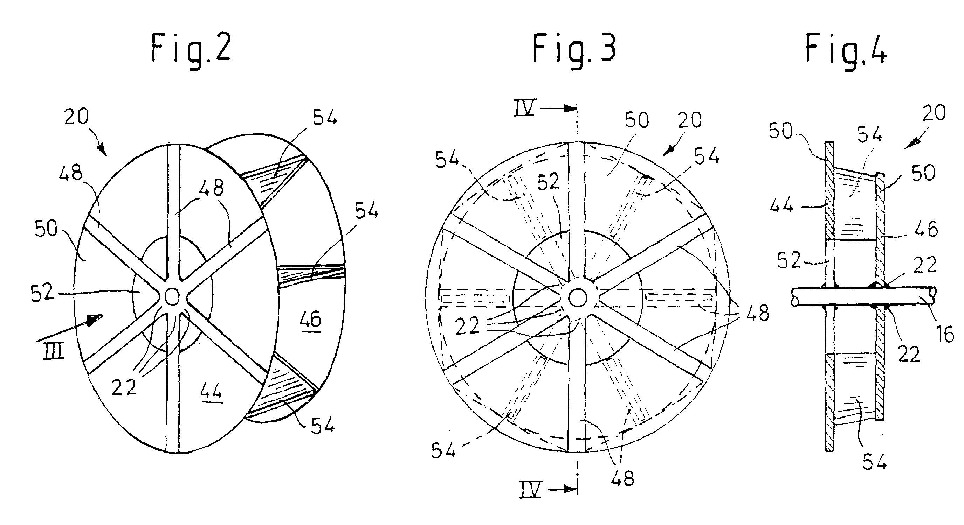

Das Laufrad 20 hat zwei im Wesentlichen parallele Stützwände 44 und 46, die zum Beispiel in einem Abstand von 2 mm über Gelenke 22 fest mit dem starren Teil 16 der Welle 12 verbunden sind. Die Stützwände 44,46 bestehen jeweils aus sechs Speichen 48. Die Winkel zwischen benachharten Speichen 48 betragen jeweils etwa 60°. Die Speichen 48 bilden jeweils ein Speichenrad, das mit einer Polymerhaut 50 bespannt ist. Die Speichen 48 und die Polymerhaut 50 bilden jeweils eine Stützrand. Die Außenkontur der beiden Stützwände 44,46 ist kreisrund und hat einen Außendurchmesser von etwa 10 mm. Alternativ kann, wie in Fig. 3 mit der Strichpunktlinie dargestellt, die Außenkante der Seitenwände zwischen den Speichen 48 verspannt sein und einen Polygonzug bilden. Die Polymerhaut 50 der vorderen Stützwand 44 hat eine den starren Teil 16 der Welle 12 umgebende kreisrunde Öffnung 52. Die Öffnung 52 hat einen Außendurchmesser von etwa 5 mm. Die Polymerhaut 50 der hinteren Stützwand 46 ist durchgehend von dem starren Teil 16 der Welle 12 bis zum äußeren radialen Ende der Stützwand 46.

Zwischen den Stützwänden 44,46 sind im Bereich der Polymerhaut 50 der vorderen Stützwand 44 radiale Flügel 54 senkrecht zu den Stützwänden 44,46 angeordnet. Die Flügel 54 sind zwischen den Stützwänden 44,46 verspannte Segel aus einer Polymerhaut. Der Radius der hinteren Stützwand 46 ist etwas geringer als der Radius der vorderen Stützwand 44. Die vordere Stützwand 44 weist in dem Betriebszustand der Pumpe einen geringen Abstand von etwa 1 mm zu der Umhüllung 24 auf. Die radial äußere Kante der hinteren Stützwand 46 hat einen größeren Abstand zu der Umhüllung 24. Dadurch wird ein Umlenkkanal 42 gebildet, durch den das Blut strömen kann, während durch den geringen Abstand zwischen vorderer Stützwand 44 und Umhüllung 24 von etwa 0,1 mm vergleichsweise wenig Blut strömen kann. Ein unerwünschter Blutrückfluss ist dadurch vermieden. Durch das Prinzip des radial fördernden Laufrades 20 sind die einzuhaltenden baulichen Toleranzen der Blutpumpe größer und die Blutpumpe ist unempfindlich gegen Fehlausrichtungen.

In dem in Fig. 1 gezeigten Betriebszustand des ersten Ausführungsbeispiels der Blutpumpe 10 strömt das Blut des im Wesentlichen axial gerichteten Blutstromes durch die vorderen Durchströmöffnungen 38 der Umhüllung 24 in die Blutpumpe 10 hinein. Die Hauptblutflussrichtungen sind in Fig. 1 durch die Pfeile angedeutet. Das Blut strömt von den Durchströmöffnungen 38 durch die Öffnung 52 in der vorderen Stützwand 44 in das Laufrad 20 hinein. Das Laufrad 20 wird von der von einem nicht dargestellten Motor angetriebenen rotierenden Welle 12 in Rotation versetzt. Die durchgehend geschlossene hintere Stützwand 46 lenkt den eintreffenden im Wesentlichen axial gerichteten Blutstrom in einen radialen Blutstrom innerhalb des Laufrades 20 in Richtung auf die Flügel 54 um. Die Rotationsbewegung der Welle 12 wird dabei von dem starren Teil 16 der Welle über die Gelenke 22 auf die Speichen 48 und schließlich auf die Flügel 54 übertragen. Die Flügel 54 schleudern das Blut durch die Rotationsbewegung des Laufrades 20 radial nach außen. Der im Laufrad radial nach außen gerichtete Blutfluss wird von der Förderwirkung der Flügel 54 verstärkt. Das radial nach außen geschleuderte Blut trifft im Bereich der Ausbauchung 30 auf die flexible Polymerhaut der Umhüllung 24 und wird von der Umhüllung 24 durch den Umlenkkanal 42 geleitet. Im Bereich des Umlenkkanals 42 wird die radiale Hauptblutflussrichtung in Richtung auf die Welle 12 umgelenkt, so dass eine axiale drallbehaftete Strömung entlang der Welle 12 erzeugt wird. Durch die in einem Abstand von ca. 0,1 mm eng an der vorderen Stützwand 44 anliegende ebene Wand 32 der Umhüllung 24 kann praktisch kein Blut zwischen Stützwand 44 und ebener Wand 32 zurückströmen.

Die im Wesentlichen trichterförmige Ausbauchung 30 leitet das von dem Laufrad 20 beschleunigte Blut in den zylindrischen Fortsatz 36 der Umhüllung 24. In dem zylindrischen Fortsatz 36 ist der Blutfluss wieder axial gerichtet. Durch die hintere Durchströmöffnung 40 strömt das Blut aus der Blutpumpe 10 heraus. Eine typische Fördermenge bei der praktischen Anwendung der Blutpumpe sind etwa 2 bis 5 I pro Minute. Die Radialpumpe benötigt zur Förderung dieser Menge eine relativ geringe Drehzahl von etwa 5.000 bis 15.000 U/min. Die Abmessungen der vom Blut durchströmten Bereiche können bei der erfindungsgemäßen Radialpumpe derart gewählt werden, dass Schädigungen des Blutes weitgehend vermieden werden.

Die Blutpumpe wird intravasal typischerweise durch die Aorta in den linken Ventrikel vorgeschoben. Hierzu ist zunächst der Außendurchmesser der Blutpumpe von etwa 10 mm durch Zusammenfalten der Pumpe zu reduzieren. Die flexible Struktur der Blutpumpe ermöglicht ein einfaches Zusammenfalten, wie in Fig. 5 gezeigt. Hierbei deuten die Pfeile die Bewegungsrichtung während des Faltvorganges an. Durch relatives Verschieben von Welle 12 und Katheter 14, z. B. indem der Katheter 14 am hinteren Ende über die Welle 12 vorgeschoben wird, wird das Laufrad 20 von der Umhüllung 24 und den Stegen 34 in Richtung auf die Welle 12 geklappt. Die Stützwände 44,46 klappen in den Gelenken 22 an die Welle, wobei die Polymerhäute 50 zwischen den Speichen 48 und die flexiblen Flügel 54 zusammenfalten. Die Speichen 48 klappen an die Welle 12, wobei sich die gegen die Speichen der vorderen Stützwand 44 versetzten Speichen der hinteren Stützwand 46 in den Zwischenräumen der Speichen der vorderen Stützwand 44 an die Welle anlegen. Durch die gegeneinander versetzte Anordnung der Speichen 48 des vorderen und des hinteren Stützrades ist der Außendtarchmesser der Blutpumpe in zusammengeklapptem Zustand mit etwa 3 mm gering-Die Blutpumpe 10 wird in zusammengeklapptem Zustand intravasal eingeführt. Zur Einführung der Blutpumpe durch Haut und Gewebe in das Blutgefäß befindet sich der Katheter 14 in einer nicht dargestellten Schleuse. Durch Zurückziehen des Katheters 14 in die Schleuse kann die Blutpumpe 10 ebenfalls zusammengefaltet werden.

Das Auffalten der Blutpumpe geschieht wie folgt: Zum Betrieb der Blutpumpe 10 wird die Welle 12 von dem nicht dargestellten Motor in Rotation versetzt. Dabei wirken Zentrifugalkräfte auf das mit der Welle 12 rotierende Laufrad 20. Diese Zentrifugalkräfte bewirken ein Aufklappen des Laufrades 20 in den Gelenken 22. Die radial äußeren Enden des Laufrades 20 drücken von innen gegen die Umhüllung 24 und falten diese automatisch auf. Durch das von dem Laufrad 20 gegen die Umhüllung 24 geförderte Blut hat die Umhüllung 24 eine stabile pralle Struktur, da der Druck im Innern der Hülle relativ zur Umgebung höher ist. Alternativ zu der dargestellten Methodik des Auffaltens erfolgt ein aktives Auffalten des Laufrads ausgehend von den Gelenken 22, da es sich bei der Stützstruktur 48,22 des Laufrades 20 um eine superelastische Metalllegierung handelt, die sich selbständig entfaltet und später durch Rotation radial stabilisiert.

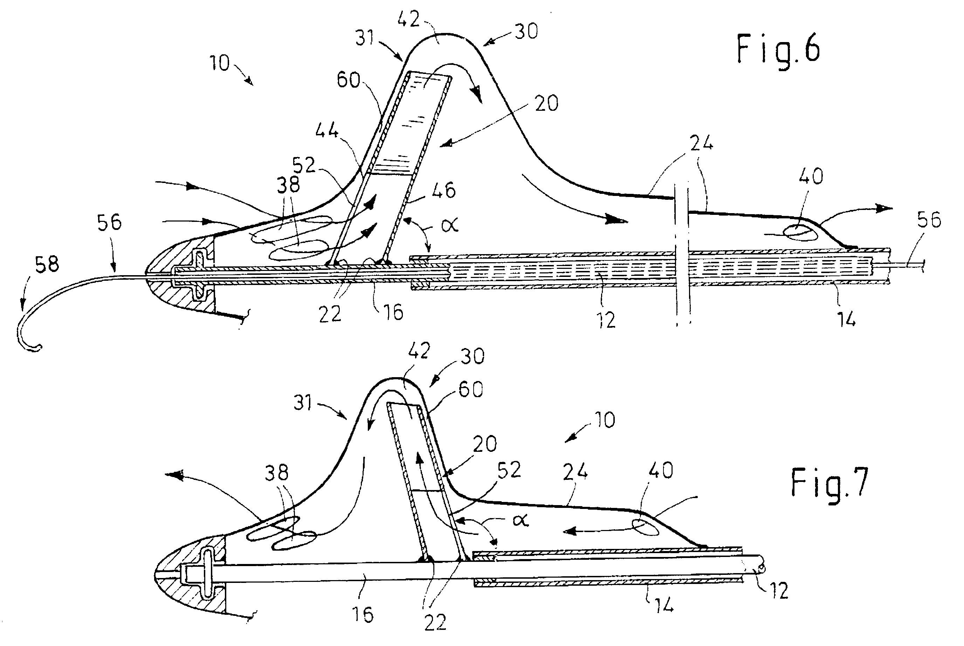

Fig. 6 zeigt ein alternatives Ausführungsbeispiel der Blutpumpe 10. Die Umhüllung 24 hat im Bereich der Ausbauchung 30 keine konzentrische ebene Wand. Das Laufrad 20 steht bei Rotation der Welle 12 zum Betrieb der Pumpe 10 nicht radial senkrecht von der Welle 12 ab, sondern kegelförmig unter einem Winkel α zwischen den Stützwänden 44,46 und der Welle 12. Der Winkel α ist kleiner als 90°. Er beträgt hier etwa 70°. Bei Rotation der Welle 12 und des starren Teils 16 der Welle stellt sich das Laufrad 20 aufgrund der wirkenden Zentrifugalkräfte automatisch auf bis der Winkel α seinen maximalen Wert im Betriebszustand er reicht. Die rotierende vordere Stützwand 44 des Laufrades 20 wird gegen die Umhüllung 24 gedrückt. Zwischen vorderer Stützwand 44 und Umhüllung 24 im Bereich der Ausbauchung 30 wird ein Kanal 60 gebildet, der durch die Rotation des Laufrades 20 ausreichend klein ist, um einen Blutrückfluss durch den Kanal 60 möglichst gering zu halten. Die Pumpwirkung der Blutpumpe ist von distal nach proximal. Durch die Schrägstellung der beiden Stützwände 44,46 des Laufrades 20 ist das Falten des Laufrades 20 durch relative axiale Verschiebung von Welle 12 und Katheter 14 erleichtert. Fig. 6 zeigt einen durch die Welle 12 geführten Führungsdraht 56, dessen Ende 58 in Form eines sogenannten "J's" geformt ist, über den die Blutpumpe 10 bis in das Herz eingeführt werden kann Zum Betrieb wird der Führungsdraht 56 entfernt.

Fig. 7 zeigt ein weiteres Ausführungsbeispiel der Blutpumpe 10, das sich von dem in Fig. 6 gezeigten Ausführungsbeispiel dadurch unterscheidet, dass das Laufrad 20 in einem Winkel α von mehr als 90° von der Welle absteht. Der Winkel α beträgt hier etwa 110°. Dadurch ist die Blutflussrichtung innerhalb der Blutpumpe 10 gegenüber Fig. 6 umgekehrt. Die Pumpwirkung der Blutpumpe ist von proximal nach distal. Das Blut strömt durch die hintere Durchströmöffnung 410 in die Blutpumpe 10 hinein und durch die vorderen Durchströmöffnungen 38 aus der Blutpumpe 10 heraus. In dem in Fig. 7 gezeigten Ausführungsbeispiel ist das Laufrad 20 in der entgegengesetzten Richtung, d. h. in Richtung auf das distale, in Fig. 7 linke Ende der Welle 12, faltbar. In Abhängigkeit von der Faltrichtung des Laufrades 20 kann die Blutpumpe 10 vorwärts fördern, d. h. von distal nach proximal wie in Fig. 6 oder - z. B. zur Rechtsherzunterstützung - rückwärts fördern, d. h. von proximal nach distal wie in Fig. 7.

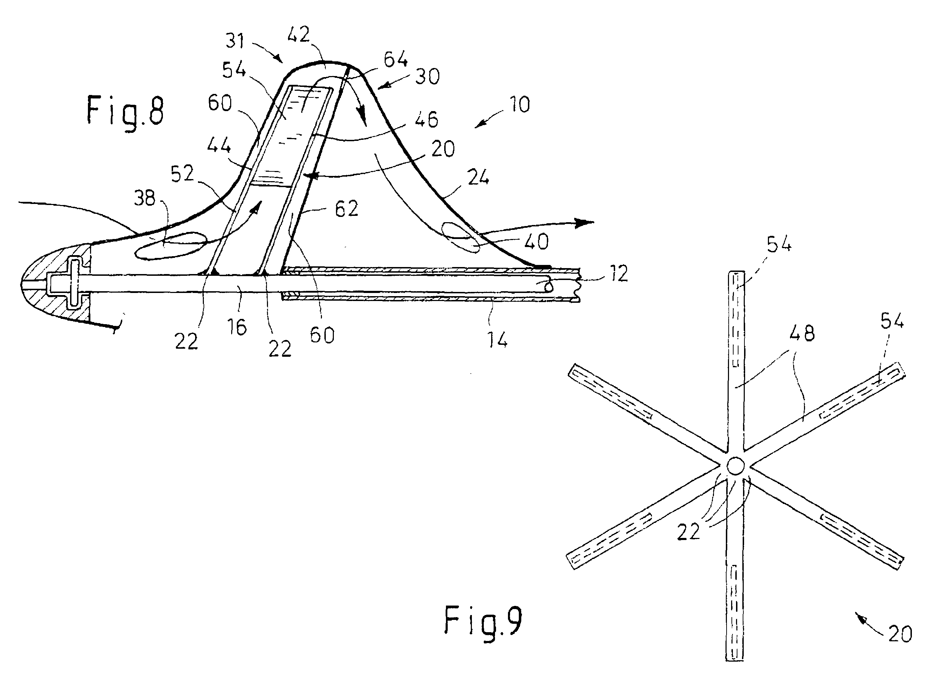

Fig. 8 zeigt ein Ausführungsbeispiel der Blutpumpe 10, bei dem zusätzlich zu der Blutpumpe nach Fig. 6 eine Trennwand 62 unmittelbar proximal der hinteren Stützwand 46 angeordnet ist. Die Trennwand 62 besteht vorzugsweise aus einer Polymerhaut, die an ihrem radial inneren Ende fest mit dem Katheter 14 verbunden ist und an ihrem radial äußeren Ende fest mit der Umhüllung 24 verbunden ist. Im Bereich des Umlenkkanals 42 hat die Trennwand Durchlassöffnungen 64, durch die das von den Flügeln 54 des Laufrades 20 beschleunigte Blut in den Bereich proximal der Trennwand 62 strömen kann. Zwischen Trennwand 62 und hinterer Stützwand 46 des Laufrades 20 wird ein zusätzlicher Kanal 60 gebildet, der im Bereich proximal des Laufrades einen unerwünschten Blutrückfluss gering hält. Das Laufrad 20 besteht, wie in Fig. 9 in der Draufsicht gezeigt, aus zwei identischen Stützwänden 44,46. Die Stützwände 44,46 werden von Speichenrädern- mit Speichen 48 gebildet. Die Speichen 48 der beiden Stützwände 44,46 sind deckungsgleich. Zwischen den Speichen 48 sind keine Polymerhäute vorgesehen. Zwischen den deckungsgleichen Speichen 48 der beiden Stützwände 44,46 sind jeweils die Flügel 54 aus segelartigen Polymerhäuten gespannt. Bei diesem Ausführungsbeispiel der Blutpumpe 10 wird die Funktion der Polymerhaut 50 der hinteren Stützwand 46 des ersten Ausführungsbeispiels durch die Trennwand 62 ersetzt. Die Struktur und der technische Aufbau der Blutpumpe 10 sind dadurch weiter vereinfacht. Auch hier kann das Laufrad 20 zusammen mit der Umhüllung 24 in Richtung auf die Welle 12 gefaltet werden, um den Außendurchmesser der Blutpumpe 10 zur einfachen intravasalen Einführung der Blutpumpe zu reduzieren.

Das Zusammenfalten der Blutpumpen gemäß den Figuren 6-8 durch relatives Verschieben von Welle 12 und Katheter 14 kann beispielsweise durch Vorschieben der Welle 12 am hinteren Ende in den Katheter 14 erfolgen. Dabei wird die Umhüllung 24 gespannt und in Richtung auf die Welle 12 zusammengezogen. Das Laufrad 20 wird von der Umhüllung 24 in Richtung auf die Welle 12 geklappt,

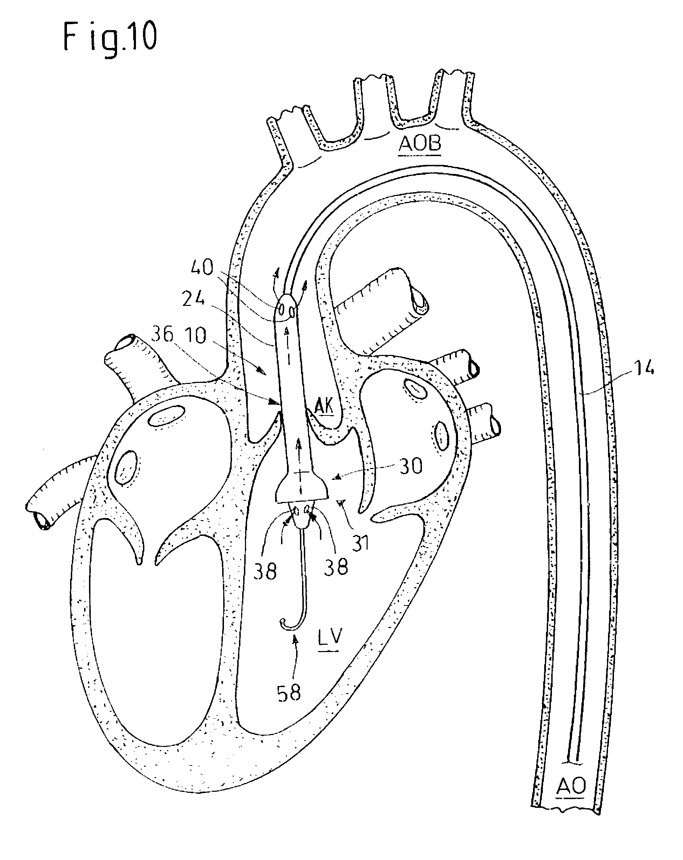

In den Figuren 10-12 sind verschiedene Arten der Platzierung der Blutpumpe im Herzen dargestellt.

Fig. 10 zeigt die Blutpumpe 10 gemäß des ersten Ausführungsbeispiels in Fig. 1. Die Blutpumpe 10 ist derart angeordnet, dass der Pumpenkopf 31 mit den distal angeordneten Durchströmöffnungen 38 innerhalb des linken Ventrikels LV liegt und der hintere Bereich der Pumpe mit den proximal angeordneten Durchströmöffnungen 40 innerhalb der Aorta AO vor dem Aortenbogen AOB liegt. Der die Welle 12 enthaltende Katheter 14 verläuft durch die Aorta AO und ist innerhalb des Aortenbogens AOB gekrümmt. Der zylindrische Fortsatz 36 der Umhüllung 24 hat eine Länge von etwa 60 bis 80 mm. Die Aortenklappe AK umschließt den zylindrischen Fortsatz 36. Die Blutpumpe 10 fördert das Blut in Vorwärtsrichtung von distal nach proximal aus dem linken Ventrikel LV in die Aorta AO. Das Blut strömt durch die distalen Durchströmöffnungen 38 in die Umhüllung 24 der Blutpumpe 10 ein. Das rotierende Laufrad beschleunigt das Blut innerhalb der Ausbauchung 30 der Umhüllung 24 und pumpt es in den zylindrischen Fortsatz 36 der Umhüllung 24. Durch die proximalen Durchströmöffnungen 40 strömt das Blut aus der Blutpumpe 10 heraus.

Fig. 10 zeigt einen Fortsatz der harten Spitze 26 in Form eines "Pig Tails", mit dem die Blutpumpe 10 an der Herzmuskelwand abgestützt wird, um einen Minimalabstand des Ansaugbereichs der Blutpumpe 1.0 von der Herzmuskelinnenwand einzuhalten.

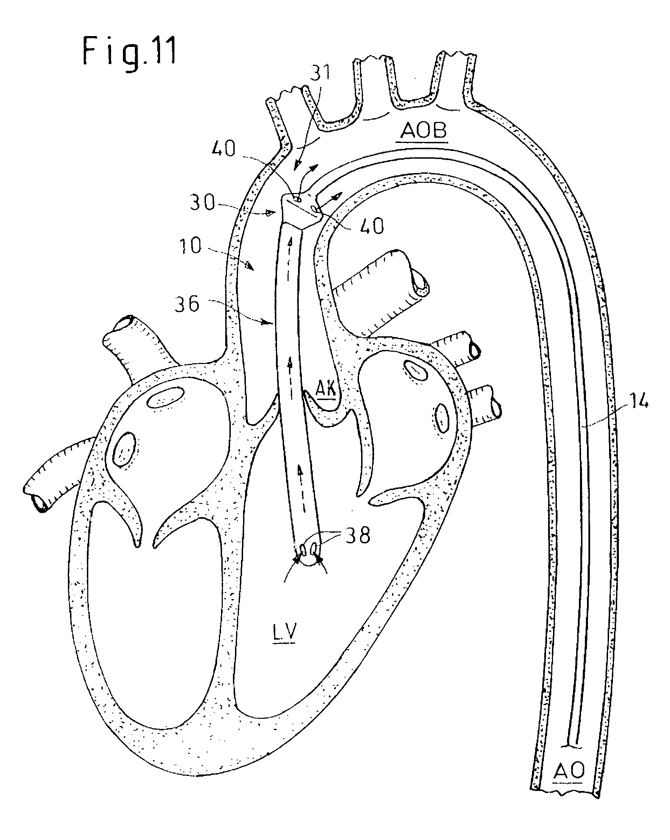

Fig. 11 zeigt die Platzierung einer proximal angeordneten Blutpumpe 10. Das distale Ende der Blutpumpe mit den Durchströmöffnungen 38 befindet sich im linken Ventrikel LV innerhalb des Herzens. Der Pumpenkopf 31 mit den proximalen Durchströmöffnungen 40 und der Ausbauchung 30 mit dem Laufrad 20 befindet sich innerhalb der Aorta AO distal des Aortenbogens AOB. Der Katheter 14 mit der Antriebswelle 12 verläuft durch den Aortenbogen AOB. Innerhalb des Aortenbogens AOB ist die flexible Welle 12 gekrümmt. Die Aortenklappe AK umschließt den zylindrischen in Förderrichtung vorgelagerten Fortsatz 36 der Blutpumpenumhüllung 24. Die Länge des zylindrischen Fortsatzes 36 beträgt etwa 60 bis 80 mm. Die Blutpumpe 10 fördert das Blut analog zur Pumpe von Fig. 10 von distal nach proximal aus dem linken Ventrikel LV in die Aorta AO. Das Blut tritt durch die distalen Durchströmöffnungen 38 in den hier gegenüber Fig. 10 vorgelagerten und radial versteiften zylindrischen Fortsatz 36 der Blutpumpenumhüllung 24 ein und wird von dem nicht dargestellten Laufrad 20 innerhalb der Ausbauchung 30 der Umhüllung 24 angesaugt und beschleunigt. Das beschleunigte Blut tritt innerhalb der Aorta AO vor dem Aortenbogen AOB durch die vorderen Durchströmöffnungen 40 aus.

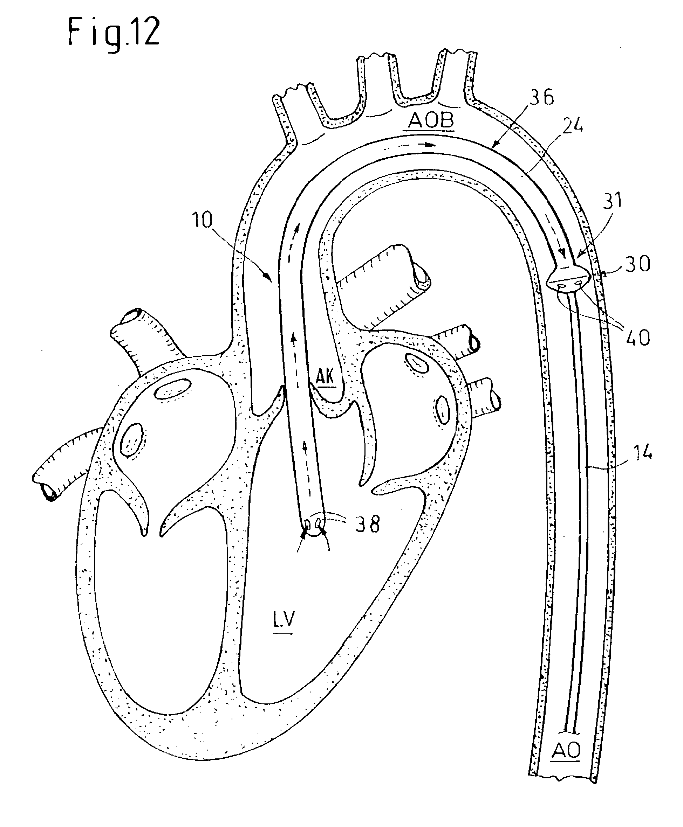

Fig. 12 zeigt ein Anwendungsbeispiel einer Blutpumpe 10 mit verlängertem zylindrischen Fortsatz 36. Der zylindrische Fortsatz 36 hat eine Länge von etwa 200 mm. Das hintere Ende der Blutpumpe 10 mit den distalen Durchströmöffnungen 38 befindet sich im linken Ventrikel LV innerhalb des Herzens. Der vorgelagerte zylindrische Fortsatz 36 verläuft von dem linken Ventrikel LV durch die Aortenklappen AK durch den Aortenbogen AOB innerhalb der Aorta AO. Proximal des Aortenbogens AOB befindet sich der Pumpenkopf 31 mit dem nicht dargestellten Laufrad 20 innerhalb der Ausbauchung 30 mit den vorderen Durchströmöffnungen 38. Das Blut tritt durch die distalen Durchströmöffnungen 38 innerhalb des linken Ventrikels LV in die Blutpumpe 10 ein und wird durch den vorgelagerten zylindrischen Fortsatz 36 durch den Aortenbogen AOB gesaugt und verlässt die Blutpumpe 10 durch die proximalen Durchströmöffnungen 40 proximal des Aortenbogens AOB. Der Katheter 14 mit der nicht dargestellten Antriebswelle 12 verläuft innerhalb der Aorta AO aber nicht durch die Krümmung des Aortenbogens AOB. Die Welle 12 ist in diesem Anwendungsbeispiel weniger stark gekrümmt. Die Gefahr einer Beschädigung der Welle durch den gekrümmten Betrieb ist reduziert.

## Patentansprüche

1. Faltbare intravasal einführbare Blutpumpe (10) mit einem Rotor mit Flügeln (54), einer durch einen Katheter (14) verlaufenden flexiblen Welle (12) zum Antreiben des Laufrades (20) und einer das Laufrad (20) umschließenden Umhüllung (24),
**dadurch gekennzeichnet,**
**dass** der Rotor ein radial förderndes Laufrad (20) ist, und dass die Umhüllung (24) im Bereich des Laufrades (20) eine ringförmige Ausbauchung (30) aufweist, wobei zwischen den radial äußeren Enden der Flügel (54) und der Umhüllung (24) ein ringförmiger Umlenkkanal (42) gebildet ist, der die radiale Strömung in axialer Richtung umlenkt.

2. Blutpumpe (10) nach Anspruch 1, **dadurch gekennzeichnet, dass** die Umhüllung (24) mit dem Katheter (14) verbunden ist und das Laufrad (20) derart mit Gelenken an der Welle (12) befestigt ist, und dass das Laufrad (20) und die Umhüllung (24) durch relatives Verschieben von Welle (12) und Katheter (14) faltbar sind.

3. Blutpumpe (10) nach Anspruch 1 oder 2, **dadurch gekennzeichnet, dass** das Laufrad (20) zwei im wesentlichen parallele Stützwände (44, 46) hat, zwischen denen flexible Segel als Flügel (54) ausgebildet sind.

4. Blutpumpe (10) nach Anspruch 3, **dadurch gekennzeichnet, dass** mindestens eine der Stützwände (44, 46) Speichen (48) enthält.

5. Blutpumpe (10) nach Anspruch 3 oder 4, **dadurch gekennzeichnet, dass** eine Stützwand (46) durchgehend ist und die andere Stützwand (44) eine die Welle (12) umgebende Öffnung (52) aufweist.

6. Blutpumpe (10) nach einem der Ansprüche 3 bis 5, **dadurch gekennzeichnet, dass** die Stützwände (44, 46) mit einem Gelenk (22) an der Welle (12) befestigt sind.

7. Blutpumpe (10) nach einem der Ansprüche 3 bis 6, **dadurch gekennzeichnet, dass** beide Stützwände (44, 46) Speichen (48) enthalten, die gegeneinander versetzt sind.

8. Blutpumpe (10) nach einem der Ansprüche 3 bis 6, **dadurch gekennzeichnet, dass** die beiden Stützwände (44, 46) aus deckungsgleichen Speichen (48) bestehen.

9. Blutpumpe (10) nach einem der Ansprüche 1 bis 8, **dadurch gekennzeichnet, dass** die Umhüllung (24) einen zylindrischen Fortsatz (36) hat, an dessen Ende Durchströmöffnungen (40) vorgesehen sind.

10. Blutpumpe (10) nach einem der Ansprüche 1 bis 9, **dadurch gekennzeichnet, dass** die Umhüllung (24) eine Nabe (26) enthält, in der die Welle (12) axial unverschiebbar gelagert ist.

11. Blutpumpe (10) nach einem der Ansprüche 1 bis 10, **dadurch gekennzeichnet, dass** die Welle (12) am vorderen Ende einen starren Teil (16) aufweist, wobei das Laufrad (20) an dem starren Teil (16) befestigt ist.

12. Blutpumpe (10) nach einem der Ansprüche 1 bis 11, **dadurch gekennzeichnet, dass** die Umhüllung (24) eine zu der Welle (12) konzentrische ebene Wand (32) aufweist, die in geringem Abstand von dem Laufrad (20) angeordnet ist.

13. Blutpumpe (10) nach einem der Ansprüche 1 bis 12, **dadurch gekennzeichnet, dass** das Laufrad (20) in zwei einander entgegengesetzten Richtungen axial zu der Welle (12) abklappbar ist.

14. Blutpumpe (10) nach einem der Ansprüche 1 bis 13, **dadurch gekennzeichnet, dass** die Umhüllung (24) auf einer Seite des Laufrades (20) eine Trennwand (62) mit einer Durchlassöffnung (64) im Bereich des Umlenkkanals (42) aufweist.

## Claims

1. A foldable intravascularly insertable blood pump (10) comprising a rotor provided with vanes (54), a flexible shaft (12) extending through a catheter (14) and adapted to drive the impeller (20), and an envelope (24) enclosing said impeller (20),
**characterized in that**
the rotor is a radially delivering impeller (20), and the envelope (24) comprises an annular bulge (30) in the region of said impeller (20), wherein between the radially outer ends of the vanes (54) and the enclosure (24) an annular deflection channel (42) is defined which deflects the radial flow in axial direction.

2. The blood pump (10) according to claim 1, **characterized in that** the envelope (24) is connected to the catheter (14) and the impeller (20) is fastened to the shaft (12) by means of hinges such that the impeller (20) and the envelope (24) are adapted to be folded by relative displacement of the shaft (12) and the catheter (14).

3. The blood pump (10) according to claim 1 or 2, **characterized in that** the impeller (20) comprises two essentially parallel supporting walls (44,46) between which flexible sails are configured as vanes (54).

4. The blood pump (10) according to claim 3, **characterized in that** at least one of the supporting walls (44,46) comprises spokes (48).

5. The blood pump (10) according to claim 3 or 4, **characterized in that** one supporting wall (46) is a continuous wall and the other supporting wall (44) comprises an opening (52) enclosing the shaft (12).

6. The blood pump (10) according to any one of claims 3 to 5, **characterized in that** the supporting walls (44,46) are fastened to the shaft (12) by a hinge (22).

7. The blood pump (10) according to any one of claims 3 to 6, **characterized in that** both supporting walls (44,46) comprise spokes (48) which are offset relative to each other.

8. The blood pump (10) according to any one of claims 3 to 6, **characterized in that** the two supporting walls (44,46) are configured of congruent spokes (48).

9. The blood pump (10) according to any one of claims 1 to 8, **characterized in that** the envelope (24) comprises a cylindrical extension (36) at the end of which flow openings (40) are provided.

10. The blood pump (10) according to any one of claims 1 to 9, **characterized in that** the envelope (24) comprises a hub (26) in which the shaft (12) is supported in an axially non-displaceable manner.

11. The blood pump (10) according to any one of claims 1 to 10, **characterized in that** the shaft (12) comprises a rigid portion (16) at its front end, wherein the impeller (20) is fastened to said rigid portion (16).

12. The blood pump (10) according to any one of claims 1 to 11, **characterized in that** the envelope (24) comprises a concentric planar wall (32) which is arranged at a small distance to the impeller (20).

13. The blood pump (10) according to any one of claims 1 to 12, **characterized in that** the impeller (20) is foldable axially to the shaft (12) in two opposite directions.

14. The blood pump (10) according to any one of claims 1 to 13, **characterized in that** the envelope (24) comprises, on one side of the impeller (20), a partition wall (62) provided with a flow opening (64) in the region of the deflection channel (42).

## Revendications

1. Pompe à sang (10) pliante, pouvant être introduite par voie intravasculaire, comprenant un rotor avec des aubes (54), un arbre flexible (12) s'étendant à travers un cathéter (14), l'arbre étant prévu pour l'entrainement de l'impulseur (20), et une gaine (24) enveloppant ledit impulseur (20),
**caractérisée en ce que**
le rotor est un impulseur (20) à refoulement radial, et que la gaine (24) comprend un renflement annulaire (30) au niveau dudit impulseur (20), un canal de déviation annulaire (42) étant formé entre les extrémités radialement extérieures des aubes (54) et la gaine (24), ledit canal déviant l'écoulement radial dans la direction axiale.

2. Pompe à sang (10) selon la revendication 1, **caractérisée en ce que** la gaine (24) est liée au cathéter (14) et que ledit impulseur (20) est attaché à l'arbre par des joints de manière que ledit impulseur (20) et la gaine (24) sont pliables par un déplacement rélatif de l'arbre (12) et du cathéter (14).

3. Pompe à sang (10) selon la revendication 1 ou 2, **caractérisée en ce que** ledit impulseur (20) comprend deux parois de support (44, 46) sensiblement parallèles, entre lesquelles des voiles flexibles sont formées comme des aubes (54).

4. Pompe à sang (10) selon la revendication 3, **caractérisée en ce qu'**au moins une des parois de support (44, 46) comprend des rayons (48).

5. Pompe à sang (10) selon la revendication 3 ou 4, **caractérisée en ce qu'**une paroi de support (46) est continue et l'autre paroi de support (44) comprend une ouverture (52) entourant ledit arbre (12).

6. Pompe à sang (10) selon l'une quelconque des revendications 3 à 5, **caractérisée en ce que** les parois de support (44, 46) sont attachées à l'arbre (12) par un joint (22).

7. Pompe à sang (10) selon l'une quelconque des revendications 3 à 6, **caractérisée en ce que** les deux parois de support (44, 46) comprend des rayons (48) décalés entre eux.

8. Pompe à sang (10) selon l'une quelconque des revendications 3 à 6, **caractérisée en ce que** les deux parois de support (44, 46) sont formées par des rayons (48) congruents.

9. Pompe à sang (10) selon l'une quelconque des revendications 1 à 8, **caractérisée en ce que** la gaine (24) comprend une extension cylindrique (36) dont l'extrémité est prévue d'ouvertures de passage (40).

10. Pompe à sang (10) selon l'une quelconque des revendications 1 à 9, **caractérisée en ce que** la gaine (24) comprend un noyau (26) dans laquelle l'arbre (12) est supporté de manière immobile dans la direction axiale.

11. Pompe à sang (10) selon l'une quelconque des revendications 1 à 10, **caractérisée en ce que** l'arbre (12) comprend une partie rigide (16) à son extrémité avant, ledit impulseur (20) étant attaché à la partie rigide (16).

12. Pompe à sang (10) selon l'une quelconque des revendications 1 à 11, **caractérisée en ce que** la gaine (24) comprend une paroi (32) plane concentrique à l'arbre (12), qui est positionnée à une distance faible dudit impulseur (20).

13. Pompe à sang (10) selon l'une quelconque des revendications 1 à 12, **caractérisée en ce que** l'impulseur (20) est apte à être basculé axialement par rapport à l'arbre (12) dans deux directions opposées l'une à l'autre.

14. Pompe à sang (10) selon l'une quelconque des revendications 1 à 13, **caractérisée en ce que** la gaine (24) comprend une paroi de séparation (62) sur un côté dudit impulseur (20), ladite paroi comprenant une ouverture de passage (64) dans la région du canal de déviation (42).
